# EUROPÄISCHE PATENTSCHRIFT

(11) **EP 2 114 857 B1**
(45) Veröffentlichungstag und Bekanntmachung des Hinweises auf die Patenterteilung: **09.01.2013**
(21) Anmeldenummer: 08717152.6
(22) Anmeldetag: 27.02.2008
(51) Int. Cl.: C07C 209/48, C07C 211/14, C07C 255/25

(54) **VERFAHREN ZUR HERSTELLUNG VON TETRAETHYLENPENTAAMIN**
METHOD FOR PRODUCING TETRAETHYLENEPENTAMINE
PROCÉDÉ DE FABRICATION DE TÉTRAÉTHYLÈNE-PENTAAMINE

(30) Priorität: 01.03.2007 EP 07103292
(43) Veröffentlichungstag der Anmeldung: 11.11.2009
(73) Patentinhaber: BASF SE, 67056 Ludwigshafen (DE)
(72) Erfinder: DAHMEN, Kirsten, 67251 Freinsheim (DE); OFTRING, Alfred, 67098 Bad Dürkheim (DE); HUGO, Randolf, 67246 Dirmstein (DE); HAHN, Thilo, 67292 Krichheimbolanden (DE); BAUMANN, Katrin, 68529 Mannheim (DE); MELDER, Johann-Peter, 67459 Böhl-Iggelheim (DE)
(86) Internationale Anmeldenummer: PCT/EP2008/052335
(87) Internationale Veröffentlichungsnummer: WO 2008/104551

(56) Entgegenhaltungen:
- EP-A- 0 222 934
- EP-A- 1 209 146
- US-A- 2 769 841
- YI LI ET AL.: "The Syntheses of Cyclic Spermine Alkaloids: Analogues of Buchnerine and Budmunchiamine C" HELVETICA CHIMICA ACTA., Bd. 86, 2003, Seiten 310-323, XP002487372 CHVERLAG HELVETICA CHIMICA ACTA. BASEL.

## Beschreibung

Die Erfindung betrifft ein Verfahren zur Herstellung von Tetraethylenpentaamin (TEPA) durch Hydrierung von Diethylentriamindiacetonitril (DETDN) an einem Katalysator. Gegebenenfalls kann DETDN auch als Bestandteil eines Aminonitrilgemisches vorliegen, das zusätzlich Diethylentriaminmonoacetonitril (DETMN) enthält.

Es ist generell bekannt, dass aliphatische Nitrile, die gegebenenfalls noch mit weiteren funktionellen Gruppen substituiert sind, in Gegenwart von Katalysatoren zu den entsprechenden Aminen hydriert werden können. Wie nachfolgend aufgezeigt, sind solche Hydrierverfahren auch für diverse Aminonitrile zur Herstellung von einigen Aminen bekannt. Bis jetzt ist jedoch nirgendwo beschrieben worden, dass auch TEPA ausgehend von dem Aminonitril DETDN oder gegebenenfalls von einem Aminonitrilgemisch enthaltend DETDN und DETMN hergestellt werden kann. Die bisher bekannten Verfahren zur Herstellung von TEPA sind jedoch, wie nachfolgend aufgeführt, mit Nachteilen verbunden.

Im Stand der Technik sind zahlreiche Verfahren zur Hydrierung der α-Aminonitrile Aminoacetonitril (AAN) und Iminodiacetonitril (IDAN) oder von β-Aminonitrilen beschrieben. So ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung oder der R₂N-C-Bindung. ""Handbook of Heterogeneous Catalytic Hydrogenation for Organic Synthesis, S. 213 - 215" zeigt die Problematik der Hydrierung von α-Aminonitrilen anhand von α-Alkylaminonitrilen oder cyclischen α-Aminonitrilen im Vergleich zu β-Aminonitrilen auf. Die bekannten Stabilitätsprobleme der α-Aminonitrile sind vermutlich der Hauptgrund dafür, warum bis heute nur die Hydrierung der α-Aminonitrile AAN oder IDAN zu EDA (Ethylendiamin) bzw. DETA (Diethylentriamin) genauer beschrieben wird. Großtechnisch werden EDA oder DETA jedoch durch die nachfolgend beschriebenen EDC- oder MEA-Verfahren hergestellt. Für höhere α-Aminonitrile ist eine entsprechende Hydrierung jedoch nicht bekannt.

In DE-A 3 003 729 wird ein Verfahren zum Hydrieren von aliphatischen Nitrilen, Alkylenoxynitrilen und Alkylenaminonitrilen zu primären Aminen in Anwesenheit eines Lösungsmittelsystems an einem Kobalt- oder Rutheniumkatalysator beschrieben. Das verwendete Lösungsmittelsystem enthält neben Wasser und Ammoniak einen Ether oder Polyether. Die als Edukte einsetzbaren Alkylenaminonitrile oder Alkylenoxynitrile sind jeweils über komplexe allgemeine Formeln definiert. Unter anderem sind als konkrete Verbindungen oder Beispiele, die zum entsprechenden Diamin hydriert werden können, Ethylendiamindipropionitril (EDDPN; auch als N,N'-Bis(cyanoethyl)-ethylendiamin bezeichnet) oder 3,3'-(Ethylendioxy)-dipropionitril aufgeführt. DE-A 3 003 729 offenbart hingegen keinen Hinweis auf die Verwendung von Einzelverbindungen von DETA-Derivaten mit Cyanomethylsubstituenten, wie DETDN oder DETMN. DETMN fällt zudem nicht unter die allgemeine Definition von Alkylenaminonitrilen gemäß diesem Dokument.

EP-A 0 382 508 beschreibt ein Verfahren zur chargenweisen Herstellung von nicht-cyclischen, aliphatischen Polyaminen durch Hydrierung von nicht-cyclischen, aliphatischen Polynitrilen in flüssiger Phase an Raney-Kobalt-Katalysatoren, vorzugsweise in Gegenwart von wasserfreiem Ammoniak. Hierbei wird eine Polynitrillösung einer Reaktionszone zugeführt, die den Raney-Kobalt-Katalysator in einer im Wesentlichen sauerstofffreien Atmosphäre enthält. Während des gesamten Reaktionszeitraums wird die Polynitrillösung mit einer Rate zugeführt, die nicht größer ist als die maximale Rate, mit der das Polynitril mit dem Wasserstoff in der Reaktionszone reagiert. Mit diesem Verfahren können Polyamine aus Polynitrilen, wie Iminodiacetonitril (IDAN), Nitrilotriacetonitril, Ethylendiamintetraacetonitril (EDTN) oder weitere nicht näher spezifizierte Verbindungen mit 2 oder mehr Cyanogruppen hergestellt werden.

EP-A 212 986 betrifft ein weiteres Verfahren, bei dem die gleichen aliphatischen Polynitrile wie in EP-A 0 382 508 in Gegenwart eines im Eduktstrom enthaltenen, flüssigen primären oder sekundären Amins an einem granularen Raney-Kobalt-Katalysators zu den entsprechenden Polyaminen hydriert werden können. Unter anderem ist als zwingend vorhandene Aminokomponente Ethylendiamin (EDA) neben zahlreichen weiteren primären oder sekundären Aminen aufgeführt.

EP-A 1 209 146 betrifft ein weiteres Verfahren zur kontinuierlichen Hydrierung von Nitrilen zu primären Aminen, wobei die jeweiligen Nitrile in flüssiger Phase an einem suspendierten, aktivierten Raney-Katalysator auf Basis einer Legierung aus Aluminium eingesetzt werden und die Reaktion in Abwesenheit von Ammoniak und basischen Alkali- oder Erdalkaliverbindungen durchgeführt wird. Als Nitrile können neben vielen anderen auch IDAN, EDTN, EDDPN oder Ethylendiaminmonopropionitril (EDMPN) zu den entsprechenden Ethylenaminen umgesetzt werden.

EP-B 0 913 388 betrifft ein Verfahren zum katalytischen Hydrieren von Nitrilen, das den Kontakt des Nitrils mit Wasserstoff in Gegenwart eines Kobaltschwammkatalysators bei Bedingungen für die Durchführung der Umwandlung der Nitrilgruppe in das primäre Amin umfasst. Der Kobaltschwammkatalysator ist zuvor mit einer katalytischen Menge von Lithiumhydroxid behandelt worden und das Verfahren wird in Gegenwart von Wasser durchgeführt. Als Nitrile eignen sich aliphatische Nitrile mit 1 bis 30 Kohlenwasserstoffatomen, unter anderem auch β-Aminonitrile wie Dimethylaminopropionitril. Ein weiteres Verfahren zur Herstellung von Polyaminen aus den entsprechenden Polynitrilen wird in DE-A 27 55 687 offenbart. In diesem Verfahren wird die Hydrierung an einem Hydrierungskatalysator in Tablettenform in Gegenwart eines den Zerfall des Katalysators inhibierenden Stabilisators durchgeführt. Als Polynitril kann unter anderem Ethylendiamindipropionitril (EDDPN) verwendet werden. Als Stabilisator eignet sich unter anderem EDA.

US-A 2006/0041170 betrifft ein Verfahren zur Herstellung von Triethylentetraamin (TE-TA), insbesondere von TETA-Salzen, und deren Verwendung als Arzneimittel. In diesem mehrstufigen Verfahren wird zunächst Ethylendiamindiacetonitril (EDDN) hergestellt. Anschließend wird EDDN an den Stickstoffatomen der beiden sekunären Aminogruppen mit Benzaldehyd oder mit Boc-Schutzgruppen (tert.-Butoxy-CarbonylGruppen) derivatisiert, beispielsweise unter Ausbildung eines (cyclischen) Imidazolidin-Derivates. Diese Derivate werden anschließend reduziert, beispielsweise durch Umsetzung mit Wasserstoff, wobei die entsprechenden Diamino-Verbindungen erhalten werden. Diese Diamino-Verbindungen werden wiederum in Gegenwart einer Säure hydrolysiert unter Erhalt des entsprechenden TETA-Salzes. Nachteilig an diesem Verfahren ist insbesondere, dass es sich um ein mehrstufiges Hydrierverfahren handelt, bei dem das eingesetzte Edukt EDDN zuerst chemisch derivatisiert werden muss, um die Hydrierung durchzuführen. Weiterhin ist nachteilig, dass zunächst TETA als Salz und nicht in der freien Basenform anfällt.

Im Stand der Technik wird somit nirgendwo beschrieben, dass DETDN oder Aminonitrilgemische enthaltend DETDN oder DETMN zur Herstellung von TEPA und gegebenenfalls weiterer Ethylenamine eingesetzt werden können. Andere Verfahren zur Herstellung von TEPA sind jedoch bekannt.

EP-A 222 934 betrifft ein Verfahren zur Herstellung von höheren Alkylenpolyaminen durch Umsetzung eines vicinalen Dihaloalkans mit einem Überschuss an Ammoniak in wässriger Phase unter Zugabe einer starken Base, wobei ein Imin-Zwischenprodukt gebildet wird, das anschließend mit einem Alkylenpolyamin unter Ausbildung des höheren Alkylenpolyamins umgesetzt wird. Als vicinales Dihaloalkan eignet sich insbesondere Ethylendichlorid (EDC oder 1,2-Dichlorethan). Als Alkylenpolyamine werden insbesondere Ethylendiamin oder höhere Ethylenamine wie DETA, aber auch TEPA und Triethylentetraamin (TETA) eingesetzt. Bei diesen Verfahren (EDC-Verfahren) fällt ein Gemisch verschiedener Ethylenamine (lineare Ethylenamine wie EDA, DETA, TETA, TEPA oder höhere Ethylenamine sowie cyclische Derivate wie Piperazin (Pip), Aminoethyl-Piperazin (AEPip) oder höhere Piperaziderivate wie Diaminoethylpiperazin (DAEPip) oder Piperazinethylethylendiamin (PEEDA)) an. Je nachdem welches Ethylenamin zu den Edukten EDC und NH₃ zugegeben wird, enthält das Reaktionsgemisch einen entsprechenden Anteil an höheren Ethylenaminen. Wenn beispielsweise gezielt TEPA hergestellt werden soll, wird den Edukten EDC und NH₃ das Ethylenamin TETA zugegeben. Auf diese Weise enthält das Produkt (Ethylenamingemisch) einen höheren Anteil an TEPA, jedoch auch die vorgenannten weiteren linearen sowie cyclischen Ethylenamine. Nachteilig an diesem Verfahren ist insbesondere, dass das Verfahren mit einer geringen Selektivität abläuft (Erhalt eines Ethylenamingemisches) und dass zuerst ein spezielles Ethylenamin hergestellt werden muss (beispielsweise DETA), welches anschließend in das Verfahren eingebracht wird, um das nächst höhere Ethylenamin (beispielsweise TETA) gezielt herzustellen bzw. die Ausbeute zu erhöhen. Dieses Verfahren stellt jedoch aufgrund der eingesetzten Edukte (Halogenalkane) und der anfallenden Salzsäure ein Korrosionsproblem sowie aufgrund der anfallenden Salze ein Umweltproblem dar.

DE-T 689 11 508 beschreibt ein alternatives Verfahren zur Herstellung von linear verlängerter Polyalkylenpolyamine wie TEPA. In diesem Verfahren wird ein bifunktioneller aliphatischer Alkohol mit einem Aminreaktanden in Anwesenheit eines Wolframhaltigen Katalysators umgesetzt. Als bifunktionaler aliphatischer Alkohol eignet sich insbesondere Monoethanolamin (MEA), als Aminreaktanden können beispielsweise EDA oder DETA eingesetzt werden. Durch dieses Verfahren werden prinzipiell Gemische aus linear verlängerten Polyalkylenpolyaminen (also Ethylenamingemische) erhalten. In diesen Ethylenamingemischen ist DETA, TETA, TEPA, Pip oder AEPip enthalten, wobei der Anteil der jeweiligen Komponente je nach eingesetzten Aminreaktanden variiert. Sofern als Aminreaktand DETA verwendet wird, wird ein Ethylenamingemisch mit hohem Anteil an TETA und TEPA erhalten. An diesem Verfahren ist nachteilig, dass das Verfahren mit einer geringen Selektivität abläuft (bezüglich der Komponenten des erhaltenen Ethylenamingemisches. Hierbei entstehen in größerer Menge Nebenprodukte wie Aminoethylethanolamin (AEEA) oder höhere Hydroxy-haltige Ethylenamine, die von geringerem wirtschaftlichem Interesse sind. Die größere Menge an anfallenden Nebenprodukten ist darin begründet, da MEA bzw. die höheren Ethanolamine (z.B. AEEA) anstelle mit dem eingesetzten Amin mit sich selbst reagieren können. Aufgrund der (statistisch) vielen Reaktionsmöglichkeiten ist die Selektivität zum linearen TEPA wegen der Koppelprodukte recht gering und nicht steuerbar. Die Synthese ist nur bei Teilumsatz möglich.

Einen Überblick über die Herstellung von Ethylenaminen liefert der SRI-Report "CEH Product Review Ethyleneamines"; SRI International, 2003; S. 53-54, in dem entsprechend der vorstehend beschriebenen Verfahren (mit den Edukten EDC oder MEA) insbesondere EDA oder DETA hergestellt werden. Dabei fallen höhere Ethylenamine wie TETA oder TEPA als Nebenprodukte an bzw. werden durch erneute Umsetzung der Edukte mit EDA oder DETA in höherer Ausbeute erhalten.

Aufgabe der vorliegenden Erfindung ist es demnach, ein einfaches und kostengünstiges Verfahren zur Herstellung von TEPA und gegebenenfalls TETA bereitzustellen.

Die Aufgabe wird gelöst durch ein Verfahren zur Herstellung von Tetraethylenpentaamin (TEPA), wobei Diethylentriamindiacetonitril (DETDN) in Gegenwart eines Katalysators und eines Lösungsmittels hydriert wird. Sofern DETDN in einem Aminonitrilgemisch vorliegt, enthaltend i) mindestens 30 Gew.-% DETDN und ii) mindestens 5 Gew.-% Diethylentriaminmonoacetonitril (DETMN), wird neben TEPA als weiteres Hauptprodukt auch TETA erhalten. Hydrieren im Rahmen der vorliegenden Erfindung bedeutet die Reaktion von DETDN oder gegebenenfalls weiterer Aminonitrile mit Wasserstoff.

Das erfindungsgemäße Verfahren hat den Vorteil, dass TEPA und gegebenenfalls die weitere Hauptkomponente TETA bei hohem Umsatz und/oder Selektivität hergestellt werden können. Die erhöhte Selektivität zeigt sich insbesondere darin, dass sowohl DETDN und gegebenenfalls DETMN selektiv hergestellt werden können als auch das eingesetzte DETDN überwiegend zu TEPA hydriert wird. Die dabei gebildeten Nebenprodukte sind hauptsächlich weitere lineare und cyclische Ethylenamine. Der Anteil an cyclischen Ethylenaminen ist im erfindungsgemäßen Verfahren im Vergleich zum EDC-Verfahren relativ gering. Die weiteren Ethylenamine sind jedoch teilweise ebenfalls interessante Wertprodukte (hauptsächlich die linearen Ethylenamine wie DETA), deren Isolierung beispielsweise in großtechnischen Verfahren lohnenswert ist.

In vorteilhafter Weise werden DETDN und gegebenenfalls DETMN vollständig oder nahezu vollständig umgesetzt. Dies ist insbesondere bei großtechnischen Verfahren von Bedeutung, da nicht umgesetztes Edukt in der Regel in den Produktionskreislauf zurückgeführt wird bzw. entsorgt werden muss. Verfahren, bei denen größere Mengen an DETDN oder DETMN nicht umgesetzt werden, sind aufgrund der hohen Instabilität dieser Aminonitrile von besonderem Nachteil. Einerseits neigen sowohl DETDN als auch DETMN sich bei höheren Temperaturen zu zersetzen, so dass die Zersetzungsprodukte nicht in den jeweiligen Kreislauf zurückgeführt werden können, andererseits kann diese Zersetzung auch mit explosionsartiger Heftigkeit verlaufen. Da im erfindungsgemäßen Verfahren die Aminonitrile vollständig umgesetzt werden können, müssen hinsichtlich der Rückführung in den Produktionszyklus keine Anstrengungen unternommen werden.

Ein weiterer Vorteil des erfindungsgemäßen Verfahrens ist, dass im Gegensatz zum EDC-Verfahren auf den Einsatz von chlorierten Kohlenwasserstoffen als Edukt verzichtet werden kann. Zudem fällt keine Salzsäure bzw. deren Salze als weiteres Reaktionsprodukt an. Die Entsorgung der vorgenannten Stoffe ist insbesondere bei großtechnischen Verfahren ein (Umwelt-)Problem. Vorteilhaft im Vergleich zum MEA-Verfahren ist, dass aufgrund der unterschiedlichen Edukte die Bildung von AEEA sowie weiterer Verbindungen mit Hydroxy-Funktion keine Rolle spielt.

Im erfindungsgemäßen Verfahren wird in einer Ausführungsform DETDN als (Haupt-)Edukt verwendet. In dieser Ausführungsform ist der Gehalt an weiteren Aminonitrilen in der Lösung, die hydriert wird, vorzugsweise auf ≤ 10 Gew.-%, insbesondere ≤ 5 Gew.-%, bezogen auf DETDN, beschränkt. In einer weiteren Ausführungsform der vorliegenden Erfindung liegt DETDN als Bestandteil eines Aminonitrilgemisches vor. Das Aminonitrilgemisch enthält neben mindestens 30 Gew.% (Gewichtsprozent) DETDN (Komponente 1), mindestens 5 Gew.-% DETMN (Komponente 2) sowie gegebenenfalls weitere Aminonitrile. DETDN ist normalerweise zu 30 bis 95 Gew.-%, bevorzugt zu 30 bis 70 Gew.-%, besonders bevorzugt zu 30 bis 50 Gew.-% im Aminonitrilgemisch enthalten. Das Aminonitrilgemisch enthält die Komponente 2 normalerweise zu 5 bis 70 Gew.-%, bevorzugt zu 30 bis 70 Gew.-%. Besonders bevorzugt enthält es die Komponente 2 zu 50-70 Gew.-%. Die vorstehenden Gewichtsprozentangaben von DETDN und DETMN sowie den weiteren Aminonitrilen beziehen sich auf die Gesamtmenge der im Gemisch enthaltenen Aminonitrile. Zusätzlich vorhandenes Wasser oder sonstige Lösungsmittel sind bei diesen Mengenangaben nicht berücksichtigt.

Unter dem Begriff "weiteres Aminonitril" soll im Rahmen der vorliegenden Erfindung jede von DETDN und DETMN verschiedene kohlenwasserstoffhaltige Verbindung verstanden werden, die mindestens drei funktionelle Gruppen enthält, wobei die funktionellen Gruppen ausgewählt sind aus einer Cyanogruppe, einer primären, einer sekundären oder einer tertiären Aminogruppe, unter der Voraussetzung, dass in der kohlenwasserstoffhaltigen Verbindung mindestens je eine Cyanogruppe und eine sekundäre Aminogruppe enthalten sind. Weiterhin soll auch Aminoacetonitril (AAN) als weiteres Aminonitril verstanden werden.

Bevorzugte weitere Aminonitrile sind ausgewählt aus Iminodiacetonitril (IDAN), Ethylendiamindiacetonitril (EDDN) oder Ethylendiaminmonoacetonitril (EDMN)

Während es sich bei den vorstehend beschriebenen weiteren Aminonitrilen um bereits literaturbekannte Verbindungen handelt und dem Fachmann Verfahren zur Herstellung dieser Einzelverbindungen bekannt sind, sind Diethylentriamindiacetonitril (DETDN) und Diethylentriaminmonoacetonitril (DETMN) neue Verbindungen, die bis jetzt noch nicht in der Literatur beschrieben worden sind. Demzufolge sind die Aminonitrile DETDN und DETMN als solche ein weiterer Gegenstand der vorliegenden Erfindung. Ebenso ist ein Verfahren zur Herstellung von DETDN oder DETMN oder eines Gemisches davon ein weiterer Gegenstand dieser Erfindung.

DETDN kann hergestellt werden durch Umsetzung von DETA mit Blausäure (HCN) und Formaldehyd. Vorzugsweise beträgt das molare Verhältnis von DETA zu Formaldehyd zu HCN 1 : 1,5 : 1,5 bis 1 : 2 : 2 [mol/mol/mol]. Dadurch dass HCN - oder gegebenenfalls FACH (wie nachfolgend dargestellt) - ohne Überschuss zu den Aminogruppen der Edukte eingesetzt wird. kann eine Selektivitätsverbesserung bei der gegebenenfalls anschließend durchgeführten Hydrierung erzielt werden.

DETDN wird vorzugsweise hergestellt durch Umsetzung von Diethylentriamin (DETA) mit Formaldehydcyanhydrin (FACH). Vorzugsweise beträgt das molare Verhältnis von DETA zu FACH 1 : 1 bis 1 : 2 [mol/mol]. Mehr bevorzugt beträgt das molare von DETA zu FACH 1 : 1,5 bis 1 : 2 [mol/mol], insbesondere ca 1 : 2 [mol/mol]. Verfahren zur Herstellung von DETA und FACH sind dem Fachmann bekannt. Vorzugsweise wird DETA im erfindungsgemäßen Verfahren in Form seiner freien Base eingesetzt, gegebenenfalls können jedoch auch Salze wie das Dihydrochlorid von DETA als Edukt verwendet werden.

Die Herstellung von FACH kann durch Umsetzung von wässrigem Formaldehyd mit Blausäure erfolgen. Vorzugsweise liegt Formaldehyd als 30 bis 50 %ige wässrige Lösung vor. Blausäure wird vorzugsweise in 90 bis 100 %iger Reinheit eingesetzt. Diese Umsetzung erfolgt vorzugsweise bei einem pH-Wert von 5,5, der vorzugsweise mit Natronlauge oder Ammoniak eingestellt wird. Für die Umsetzung kann bei Temperaturen von 20 bis 70°C beispielsweise im Schlaufen- und/oder Rohrreaktor erfolgen.

Anstelle von aufgereinigter Blausäure (HCN) kann auch HCN-Rohgas in wässriger Formaldehyd-Lösung unter den oben genannten Bedingungen zu FACH chemisorbiert werden. Das HCN-Rohgas wird vorzugsweise durch Pyrolyse von Formamid hergestellt und enthält neben Wasser insbesondere geringe Anteile an Ammoniak.

Gegebenenfalls kann die erhaltene wässrige FACH-Lösung durch schonende Vakuumeindämpfung, beispielsweise mit einem Fallfilm- oder Dünnschichtverdampfer, aufkonzentriert werden. Vorzugsweise erfolgt eine Aufkonzentrierung auf eine 50-80 %ige FACH-Lösung. Vor der Aufkonzentrierung ist eine Stabilisierung der FACH-Lösung durch Erniedrigung des pH-Wertes auf ≤ 4, bevorzugt auf ≤ 3 vorteilhaft, beispielsweise durch Säurezugabe, zum Beispiel durch Zugabe von Phosphorsäure oder vorzugsweise von Schwefelsäure.

Alternativ kann DETDN auch durch Umsetzung eines Diethylentriamin-Formaldehyd-Addukts (DTFA) mit Blausäure (HCN) hergestellt werden, wobei das molare Verhältnis von DTFA zu HCN vorzugsweise 1 : 1,5 bis 1 : 2 [mol/mol] beträgt. Weiterhin kann DETA auch mit Formaldehyd und Blausäure zeitgleich (parallel) umgesetzt werden. Denkbar ist auch, dass zunächst ein Gemisch aus Formaldehyd und Blausäure hergestellt wird, das anschließend mit DETA umgesetzt wird. Vorzugsweise beträgt bei diesen Verfahrensalternativen das molare Verhältnis von DETA zu der oder den anderen Edukten 1 : 1,5 bis 1 : 2 [mol/mol].

Die Reaktion kann bei 10 bis 90°C, vorzugsweise bei 30 bis 70°C sowie bei Normal- oder Überdruck durchgeführt werden. Vorzugsweise wird die Reaktion in einem Rohrreaktor oder einer Rührkesselkaskade durchgeführt.

Vorzugsweise erfolgt die Herstellung von DETDN in einem Lösungsmittel, insbesondere in Gegenwart von Wasser. Gegebenenfalls können neben Wasser noch weitere mit Wasser mischbare Lösungsmittel verwendet werden. Die Gegenwart von Alkoholen, insbesondere Methanol ist jedoch weniger vorteilhaft.

Bei der Herstellung von DETDN (Hauptprodukt) fallen in dieser Ausführungsform des erfindungsgemäßen Verfahrens als wichtigstes Nebenprodukt DETMN an. Je nach Wahl der entsprechenden Verfahrensparameter (beispielsweise Edukt, Temperatur, Lösungsmittel oder Druck) kann das erfindungsgemäße Verfahren so gesteuert werden, dass der Anteil an DETDN im Reaktionsprodukt variiert und DETMN nicht als Nebenprodukt, sondern als zweites Reaktionshauptprodukt anfällt. In dieser Ausführungsform der vorliegenden Erfindung wird somit ein Aminonitrilgemisch hergestellt, das neben DETDN auch DETMN (als Hauptprodukt) enthält. Vorzugsweise werden dabei Aminonitrilgemische gemäß der vorstehend definierten Konzentrationsangaben hergestellt.

Vorzugsweise wird eine Erhöhung des Anteils an DETMN im Aminonitrilgemisch dadurch erzielt, dass in den vorstehend beschriebenen Synthesevarianten in den angegebenen Parameterbereichen jeweils ein geringerer molarer Anteil an FACH, Formaldehyd oder Blausäure verwendet wird. So wird beispielsweise zur Erhöhung des DETMN-Anteils ein molares Verhältnis von DETA zu FACH von 1 : 1,5 bis 1 : 1,8 [mol/mol] verwendet. Um möglich reines DETMN herzustellen, so dass DETDN nur als Nebenprodukt vorliegt, wird der molare Anteil an FACH weiter reduziert, vorzugsweise zu einem Verhältnis von DETA zu FACH von ca. 1 : 1 [mol/mol].

Weiterhin kann ein Aminonitrilgemisch, das einen geringeren Anteil an DETMN enthält, beispielsweise ≤ 10 Gew.-%, vorzugsweise 5 bis 10 Gew.-%, insbesondere ≤ 1 Gew.-% in einer Ausführungsform der vorliegenden Erfindung durch Umsetzung von DETA mit einem möglichst hohen molaren Anteil an FACH hergestellt werden. Hierbei wird vorzugsweise eine wässrige Lösung von ≥ 40 Gew.-% an FACH oder reines FACH eingesetzt. Das molare Verhältnis von DETA zu FACH beträgt in diesem Fall vorzugsweise 1 : 2 [mol/mol].

Im Anschluss an die Herstellung von DETDN oder DETMN oder Gemischen enthaltend DETDN und DETMN können die Einzelverbindung nach dem Fachmann bekannten Methoden isoliert werden. Vorzugsweise erfolgt eine Isolierung durch Kristallisation. Kristallisationsverfahren als solche sind dem Fachmann bekannt. In einer Ausführungsform der vorliegenden Erfindung wird DETDN vorzugsweise direkt im Anschluss an dessen Herstellung in der Regel ohne weitere Aufreinigungsschritte der Hydrierung unterzogen. Vorzugsweise liegt DETDN dabei als Bestandteil eines der vorstehend beschriebenen Aminonitrilgemische vor, das zusätzlich DETMN enthält. Vor der Hydrierung können gegebenenfalls ein oder mehrere der nachfolgend aufgeführten Aufreinigungsschritte durchgeführt werden. Vorzugsweise erfolgt die Hydrierung mit Ausnahme der Wasserabreicherung und/oder Leichtsiederabtrennung ohne zusätzliche Zwischenschritte im Anschluss an die Herstellung von DETDN.

### i) Leichtsieder-Abtrennung

In einer Ausführungsform der vorliegenden Erfindung werden vor der Hydrierung die Leichtsieder aus dem Reaktionsprodukt von Schritt a) abgetrennt. Sofern zur Herstellung von DETDN und gegebenenfalls DETMN FACH verwendet wird, kann die Leichtsiederabtrennung bereits vor der Umsetzung von FACH mit DETA erfolgen.

Vorzugsweise wird Blausäure (HCN) als Leichtsieder abgetrennt. HCN kann dabei auch als Zersetzungsprodukt von FACH auftreten. Weiterhin kann an dieser Stelle eventuell vorhandener Ammoniak abgetrennt werden. Vorzugsweise erfolgt die Abtrennung durch Destillation, beispielsweise in Form einer Sambay-Destillation ("Chemie Ingenieur Technik, Vol. 27, S. 257-261). Gegebenenfalls kann das Reaktionsgemisch auch mit Stickstoff gestrippt werden.

### ii) Abreicherung von Wasser

Wasser kann entweder zusammen mit den Leichtsiedern oder, bevorzugt, nach der Leichtsiederabtrennung ganz oder teilweise abgereichert werden. Vorzugsweise erfolgt die Abreicherung des Wassers als Destillation. Dies kann ein- oder mehrstufig in einem Verdampfer bzw. einer Verdampferkaskade erfolgen, wobei von Stufe zu Stufe unterschiedliche Drücke bzw. Temperaturen eingestellt werden können. Die Wasserabtrennung kann auch in einer Destillationskolonne erfolgen. Bevorzugt erfolgt die Wasserabtrennung im Vakuum. Das verbleibende Aminonitril oder Aminonitrilgemisch kann noch Reste an Wasser und Leichtsiedern enthalten. Bevorzugt ist ein Restwassergehalt von mindestens 10 Gew.-%. Die Leichtsieder sind dann nur noch in geringen Spuren enthalten.

Generell kann jede Art/Qualität von DETDN und gegebenenfalls von DETMN sowie von weiteren Aminonitrilen bei der Hydrierung eingesetzt werden. Bevorzugt werden die entsprechenden Aminonitrile in Form ihrer wässrigen Lösung eingesetzt. Wie vorstehend bereits aufgeführt, können DETDN sowie DETMN vor dem Einsatz im erfindungsgemäßen Verfahren nach dem Fachmann bekannten Methoden aufgereinigt werden. Sofern DETDN im erfindungsgemäßen Verfahren in einem Aminonitrilgemisch enthaltend DETDN oder DETMN und gegebenenfalls weitere Aminonitrile eingesetzt wird, können die einzelnen Komponenten dieses Aminonitrilgemisches getrennt voneinander synthetisiert werden und vor dem Einsatz im erfindungsgemäßen Verfahren in den entsprechenden Mengen zum Aminonitrilgemisch vereint werden.

DETDN ist bei Raumtemperatur ein Feststoff, ebenso DETMN. Folglich wird die Hydrierung des erfindungsgemäßen Verfahrens in Gegenwart von einem Lösungsmittel wie einem organischen Lösungsmittel und/oder Wasser durchgeführt. Vorzugsweise wird Wasser als Lösungsmittel verwendet, gegebenenfalls können auch Gemische aus Wasser und organischen Lösungsmitteln wie Ether, insbesondere THF, verwendet werden. Die zusätzliche Verwendung eines organischen Lösungsmittels (d.h. einer inerten organischen Verbindung) neben Wasser erweist sich jedoch als vorteilhaft, da dadurch eine Stabilisierung der einzelnen Komponenten des wässrigen Aminonitrilgemisches, insbesondere in Gegenwart der resultierenden Amine, erzielt werden kann. Außerdem lässt sich durch die Verwendung von organischen Lösungsmitteln ein Spüleffekt (Reduzierung der Spülcyclen, Verminderung der Katalysatorausschleusung) an dem eingesetzten Katalysator erzielen, wodurch dessen Standzeit erhöht bzw. dessen Verbrauch erniedrigt (längere Katalysatorlebensdauer) sowie die Katalysatorbelastung verbessert werden kann.

Ein geeignetes Lösungsmittel, welches ein oder mehrere Komponenten umfassen kann, sollte bevorzugt folgende Eigenschaften aufweisen:
(a) das Lösungsmittel sollte stabilisierend auf DETDN oder gegebenenfalls DETMN wirken, insbesondere deren Zersetzung bei den herrschenden Temperaturen verhindern;
(b) das Lösungsmittel sollte eine gute Wasserstofflöslichkeit zeigen;
(c) das Lösungsmittel sollte bei den Reaktionsbedingungen inert sein;
(d) das Reaktionsgemisch (DETDN, gegebenenfalls DETMN sowie Wasser oder organisches Lösungsmittel) sollte unter Reaktionsbedingungen einphasig sein;
(e) die Lösemittelauswahl sollte im Hinblick auf eine bevorzugt destillative Abtrennung des Produktes im Anschluss an die Hydrierung aus dem Produktstrom erfolgen. Wobei energie- oder apparativ-aufwändige (z.B. engsiedende Gemische oder schwierig zu trennende Azeotrope) Trennungen zu vermeiden sind.
(f) das Lösungsmittel sollte gut von den Produkten abtrennbar sein, d.h. die Siedetemperatur sollte sich hinreichend von der der Produkte unterscheiden. Hierbei wird eine niedrigere Siedetemperatur als die der Produkte bevorzugt.

Mögliche Lösungsmittel (neben Wasser) sind organische Lösungsmittel, beispielsweise Amide, wie N-Methylpyrrolidon (NMP) und Dimethylformamid (DMF), aromatische und aliphatische Kohlenwasserstoffe, wie Benzol und Xylol, Alkohole wie Methanol, Ethanol, Propanol, Isopropanol, Butanol, Isobutanol, sekundäres Butanol und tertiäres Butanol, Amine, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran. In einer weiteren bevorzugten Ausführungsform werden Alkohole, insbesondere Methanol, als organisches Lösungsmittel verwendet.

Das organische Lösungsmittel wird im Gewichtsverhältnis zu dem eingesetzten Aminonitril (DETDN und gegebenenfalls DETMN) von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate bzw. Verweilzeit eingestellt werden kann. Es ist bevorzugt, das Aminonitril zu 10 bis 50 Gew.% mit dem Lösungsmittel bzw. Gemisch zu vermischen. Bezogen auf die besonders bevorzugten Lösungsmittel Methanol bzw. Tetrahydrofuran ist es beispielsweise vorteilhaft, das Aminonitril zu 20 bis 40 Gew.-% bezogen auf das Lösungsmittel einzusetzen.

Sofern Wasser vorhanden ist, liegt der Anteil an Wasser in der Lösung in einem Bereich von 0 bis 60 Gew.-%, vorzugsweise bei 10 bis 30 Gew.-%. Die Mengenangaben des Wassers beziehen sich dabei auf das Aminonitril-Wasser-Gemisch. Gegebenenfalls können in der Lösung, in der die Hydrierung durchgeführt wird, zusätzliche Additive enthalten sein. Als Additive kommen prinzipiell Hydroxide wie Alkalimetallhydroxide, Alkoholate, Amide, Amine sowie gegebenenfalls Ammoniak in Frage. Vorzugsweise eignen sich als Additive Amine, bevorzugt das Amin EDA und Ammoniak, insbesondere EDA. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren unter Zusatz von Additiven durchgeführt.

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoffenthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.% ZrO₂, 50 Gew.-% NiO, 17 Gew.-% CuO und 1,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als ,Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/AI-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1 - 30 Gew.-% Al, besonders 2 - 12 Gew.-% Al, ganz besonders 3 - 6 Gew.-% Al, 0 - 10 Gew.-% Cr, besonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew.-% Ni, insbesondere 1 - 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2-6 Gew.-%, Co: ≥ 86 Gew.-%, Fe: 0-1 Gew.-%, Ni: 1-4 Gew.-%, Cr: 1,5 - 3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch
1 - 30 Gew.-% Al, besonders 2 - 20 Gew.-% Al, ganz besonders 5 - 14 Gew.-% Al,
0 - 10 Gew.-% Cr, besonders 0,1 - 7 Gew.-% Cr, ganz besonders 1 - 4 Gew.-% Cr, und/oder
0 - 10 Gew.-% Fe, besonders 0,1 - 7 Gew.-% Fe, ganz besonders 1 - 4 Gew.-% Fe, wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden. Dieser Katalysator weist folgende Zusammensetzung auf
Al: ≤14 Gew.%, Ni: ≥ 80 Gew.%, Fe: 1-4 Gew.%, Cr: 1-4 Gew.%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierlich entnommen, ex situ regeneriert und zurückgeführt.

Die Temperaturen, bei denen das erfindungsgemäße Verfahren durchgeführt wird, liegen in einem Bereich von 40 bis 150°C, bevorzugt von 70 bis 140°C, insbesondere bei 80 bis 130°C.

Der bei der Hydrierung herrschende Druck liegt im Allgemeinen bei 5 bis 300 bar, bevorzugt bei 30 bis 250 bar, besonders bevorzugt bei 40 bis 160 bar.

In einer bevorzugten Ausführungsform wird DETDN beziehungsweise das Aminonitrilgemisch enthaltend DETDN und DETMN mit einer Rate der Hydrierung zugeführt, die nicht größer ist als die Rate, mit der DETDN und gegebenenfalls die übrigen Komponenten des Aminonitrilgemisches mit Wasserstoff bei der Hydrierung reagiert.

Die Zuführrate ist bevorzugt so einzustellen, dass quasi Vollumsatz erreicht wird. Dieses wird durch Temperatur, Druck, Art des Gemisches, Menge und Art des Katalysators, des Reaktionsmediums, Durchmischungsgüte des Reaktorinhalts, Verweilzeit etc. beeinflusst.

Im erfindungsgemäßen Verfahren werden ein (oder mehrere) Lösungsmittel verwendet, wobei das Lösungsmittel zunächst vollständig mit DETDN oder dem Aminonitrilgemisch vermischt wird. Die erhaltene Lösung, die gegebenenfalls auch Additive enthalten kann, wird anschließend in das den Katalysator enthaltende Reaktionsgefäß zugeführt. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird. Bei kontinuierlichen Verfahren kann eine Teilmenge des Lösungsmittels auch separat von der Lösung, die DETDN, das Lösungsmittel und gegebenenfalls das Additiv enthält, in das Reaktionsgefäß zugegeben werden. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird. Besonders bevorzugt wird das Aminonitril in wässriger Lösung zugefahren und das organische Lösungsmittel separat zudosiert.

Das erfindungsgemäße Verfahren zur Herstellung von TEPA durch Hydrierung von DETDN kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrils und des Katalysators mit dem gasförmigen Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, bzw. in einer Kaskade derartiger gleicher oder verschiedener Reaktoren durchgeführt werden. Für die Hydrierung an einem Festbettkatalysator sind Rohrreaktoren aber auch Rohrbündelreaktoren denkbar.

Im Fall eines Festbettkatalysator wird in Sumpf- oder Rieselfahrweise mit dem Aminonitril beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmantel oder außenliegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeüberträgers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Das erfindungsgemäße Verfahren liefert durch die Hydrierung als Hauptprodukt TEPA (1. Fall) sowie weitere Ethylenamine als Nebenkomponenten. Sofern im erfindungsgemäßen Verfahren ein Aminonitrilgemisch enthaltend DETDN und DETMN eingesetzt wird, erhält man ein Ethylenamingemisch, das als Hauptkomponenten TEPA und TETA (2. Fall) sowie als Nebenkomponenten weitere Ethylenamine enthält. Das erfindungsgemäße Verfahren wird im nachfolgenden Schema 1 anhand des zweiten Falles verdeutlicht, bei dem ein Aminonitrilgemisch enthaltend DETDN und DETMN gemeinsam hergestellt und anschließend hydriert wird.

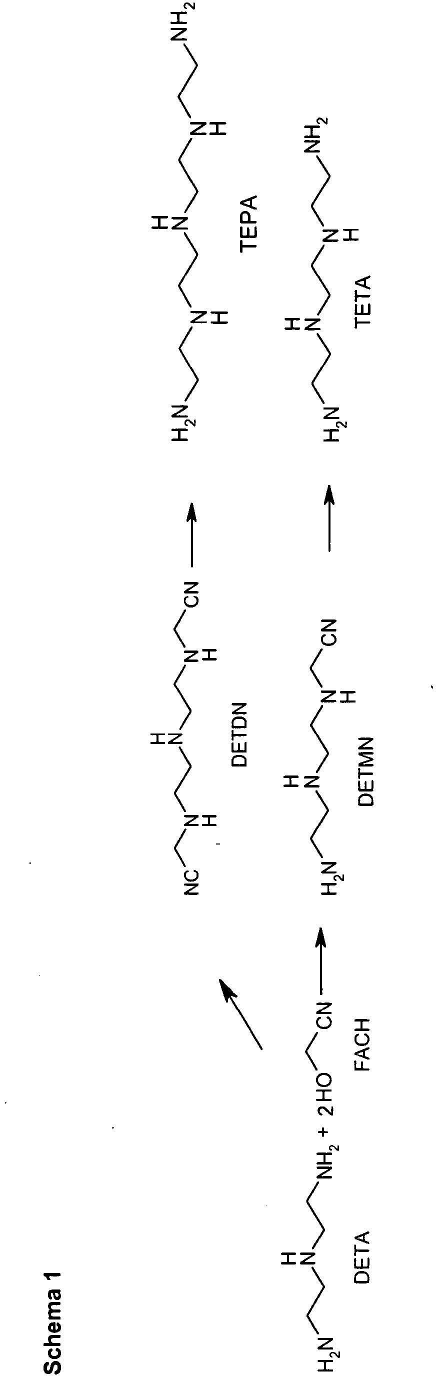

Im zweiten Fall wird der Begriff "Ethylenamingemisch" deswegen verwendet, weil das Reaktionsprodukt zwei Hauptkomponenten (TEPA und TETA) enthält, während dem ersten Fall nur ein Hauptprodukt (TEPA) vorliegt. Die nachfolgend aufgeführten Nebenprodukte werden folglich für die Begriffsdefinition in diesen beiden Fällen nicht berücksichtigt.

Im ersten Fall erhält man TEPA mit einer Selektivität von vorzugsweise ≥ 75 Gew.%, insbesondere ≥ 90 Gew.-%, bezogen auf die eingesetzte Menge an DETDN.

Unter dem Begriff "weiteres Ethylenamin" soll im Rahmen der vorliegenden Erfindung jede von TEPA (1. Fall) und von TETA und TEPA (2. Fall) verschiedene kohlenwasserstoffhaltige Verbindung verstanden werden, die mindestens zwei Ethyleneinheiten und mindestens zwei funktionelle Gruppen enthält, wobei die funktionellen Gruppen ausgewählt sind aus einer primären, einer sekundären oder einer tertiären Aminogruppe. Als weiteres Ethylenamin sollen im Rahmen der vorliegenden Erfindung auch cyclische Verbindungen wie beispielsweise Piperazin (Pip) sowie dessen Derivate verstanden werden. Ebenfalls soll Ethylendiamin (EDA) als weiteres Ethylenamin aufgefasst werden. DETA und Pip werden auch als C₄-(Neben)-Produkte und AEPip und TETA als C₆-(Neben)-Produkte bezeichnet.

Bevorzugte weitere Ethylenamine sind ausgewählt aus TETA (jeweils nur 1. Fall) sowie Diethylentriamin (DETA), Piperazin (Pip), Aminoethylenpiperazin (AE-Pip) oder Diaminoethylpiperazin (DAEPip) sowie Piperazinylethylethylendiamin (PEEDA).

Im Anschluss an die Hydrierung kann das erhaltene Produkt (TEPA oder Ethylenamingemisch) gegebenenfalls weiter aufgereinigt werden, beispielsweise indem das Lösungsmittel und/oder der Katalysator nach dem Fachmann bekannten Methoden abgetrennt werden. Insbesondere können die Hauptprodukte (TEPA und gegebenenfalls TETA) gemeinsam oder einzeln nach dem Fachmann bekannten Methoden aus dem Reaktionsprodukt isoliert werden. Sofern die beiden Hauptprodukte gemeinsam isoliert werden, beispielsweise durch eine Destillation, können sie anschließend in die beiden Einzelprodukte isoliert werden. Letztendlich erhält man somit reines TEPA und reines TETA. Sonstige Verunreinigungen, Nebenprodukte oder weitere Ethylenamine wie DETA oder Pip können ebenfalls mit dem Fachmann bekannten Methoden aus dem jeweiligen Produkt abgetrennt werden. Alternativ kann auch eine gemeinsame Isolierung von TETA mit den cyclischen Produkten PEEDA bzw. DEAPip durchgeführt werden.

In einer bevorzugten Ausführungsform wird das erfindungsgemäße Verfahren unter Verwendung von Tetrahydrofuran oder Methanol als Lösungsmittel durchgeführt. Die Temperatur bei der Hydrierung beträgt vorzugsweise 80 bis 140°C, der Druck vorzugsweise 40 bis 160 bar. Vorzugsweise wird die Hydrierung in Anwesenheit von EDA und/oder gegebenenfalls von Ammoniak durchgeführt.

Die nachfolgenden Beispiele verdeutlichen das erfindungsgemäße Verfahren. Die Anteile sind in Gew.-% angegeben, sofern nicht anders aufgeführt. Ein mitgeführter interner Standard, Diethylenglykoldimethylether (DEGDME), erlaubt eine Quantifizierung des Produktes durch Bestimmung der eventuell gebildeten flüchtigen Zersetzungsbestandteile. Die Quantifizierung erfolgt mittels Gaschromatographie (GC), wobei den jeweils entnommenen Proben zur Homogenisierung Methanol zugegeben wird.

### Beispiele:

### Allgemeine Vorschrift zur Synthese von Formaldehydcyanhydrin (FACH)

### Variante a)

In einem 61-Reaktionsgefäß mit Propellerrührer werden 6000 g (60 mol) Formaldehyd (30 %) vorgelegt und mit Natronlauge (1 mol/l) ein pH-Wert von 5.5 eingestellt. Innerhalb 2.5 Stunden werden 1661 g (61.2 mol) Blausäure über ein beheiztes U-Rohr unterhalb des Rührers gasförmig eindosiert, wobei die Reaktionstemperatur bei 30°C und der pH-Wert bei 5.5 gehalten wird. Nach 30 Minuten Nachrührzeit wird der pH-Wert mit Schwefelsäure (50 %ig) auf 2.5 gestellt. Über Liebig-Titration wird der entsprechende Gehalt ermittelt.

### Variante b)

In einem 61-Reaktionsgefäß mit Propellerrührer werden 7000 g (70 mol) Formaldehyd (30 %) vorgelegt und mit Natronlauge (1 mol/l) ein pH-Wert von 5.5 eingestellt. Innerhalb 3 Stunden werden 1938 g (71,4 mol) Blausäure über ein auf 50 °C beheiztes U-Rohr unterhalb des Rührers gasförmig eindosiert, wobei die Reaktionstemperatur bei 30 °C und der pH-Wert bei 5.5 gehalten wird. Nach 10 Minuten Nachrührzeit wird der pH-Wert mit Schwefelsäure (50 %ig) auf 2.5 gestellt. Um Leichtsieder, insbesondere Blausäure abzutrennen, wird der Reaktionsaustrag einer Sambaydestillation (wie in "Chemie Ingenieur Technik, Vol. 27, S. 257-261 beschrieben) (1 mbar, 30 °C) unterzogen. Über Liebig-Titration wird der entsprechende Gehalt ermittelt und gegebenenfalls durch Zugabe von Wasser ein Gehalt von 43-44 % bzw. 67 % FACH eingestellt.

### Beispiel 1:

### Formaldehydcyanhydrin

FACH wird nach der allgemeinen Vorschrift nach Variante b) hergestellt.

### Diethylentriamindiacetonitril

In einem 21-Reaktionsgefäß werden 165 g (2.2 mol) DETA vorgelegt und unter Eiskühlung bei einer Temperatur von maximal 30 °C innerhalb von 2 Stunden 511 g (4 mol) FACH (44.6 %) zugetropft. Nach 3 Stunden Nachrührzeit wird die leicht gelbe Lösung abgefüllt. Der Umsatz FACH beträgt laut Liebig Titration 99.3 %. Der Reaktionsansatz enthält 0.09 % freie Blausäure (bestimmt durch Volhard-Titration). Durch Titration wird eine DETDN-Ausbeute von 90,2 % bezogen auf eingesetztes FACH erhalten. DETMN lässt sich durch Titration nicht bestimmen. Unter der Annahme, dass aus umgesetztem Diethylentriamin, welches nicht zu DETDN reagiert, DETMN gebildet wird, ergibt sich als Gesamt-Aminonitril-Ausbeute 93,5 % und somit eine Ausbeute an DETMN von 3 %.

### Tetraethylenpentaamin - TEPA

a) Die erhaltene Ware (DETDN-Herstellung) wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-DETDN-Lösung, 13,8g eines internen Standards sowie 10g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 12% PEEDA sowie 67% TEPA. Zusätzlich werden 9% an C₄-und C₆- Produkten (Pip, DETA , TETA und AEPIP) erhalten.
   Durch den Überschuss an DETA in der DETDN-Synthese wird DETMN auch gebildet, welches zu den C₆-Produkten TETA und AEPIP hydriert wird.
b) Die erhaltene Ware (DETDN-Herstellung) wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-DETDN-Lösung, 13,8g eines internen Standards sowie 10g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 6% PEEDA sowie 76% TEPA. Desweiteren werden 14% an C₄- und C₆-Produkten erhalten.
   Durch die EDA-Zugabe wird mehr lineares TEPA gebildet. Ebenfalls zeigt sich ein Anstieg an C₄- und C₆-Produkten, der auf EDA-Kondensation beruht.
c) Die erhaltene Ware (DETDN-Herstellung) wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-DETDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 6% PEEDA sowie 82% TEPA. Zusätzlich wurden 10% an C₄- und C₆-Produkten erhalten.

Im Vergleich zu den Beispielen 1a und 1b wird auf eine zusätzliche Zugabe an Wasser verzichtet, was sich positiv auf die TEPA-Selektivität auswirkt.

## Patentansprüche

1. Verfahren zur Herstellung von Tetraethylenpentaamin (TEPA), wobei Diethylentriamindiacetonitril (DETDN) in Gegenwart eines Katalysators und eines Lösungsmittels hydriert wird.

2. Verfahren gemäß Anspruch 1 wobei DETDN in einem Aminonitrilgemisch vorliegt enthaltend i) mindestens 30 Gew.-% DETDN und ii) mindestens 5 Gew.-% Diethylentriaminmonoacetonitril (DETMN).

3. Verfahren gemäß Anspruch 1 oder 2, **dadurch gekennzeichnet, dass** ein Raney-Katalysator eingesetzt wird, bevorzugt ein Raney-Nickel- oder ein Raney-Kobalt-Katalysator, insbesondere ein Raney-Kobalt-Skelett-Katalysator, der aus einer Co/Al-Legierung durch Laugung mit wässriger Alkalimetallhydroxid-Lösung erhalten wird und der als Promotor mindestens eines der Elemente Fe, Ni oder Cr enthält.

4. Verfahren gemäß einer der Ansprüche 1 bis 3, **dadurch gekennzeichnet, dass** das Lösungsmittel Wasser und/oder ein organisches Lösungsmittel, insbesondere Tetrahydrofuran oder Methanol, ist.

5. Verfahren gemäß einem der Ansprüche 1 bis 4, **dadurch gekennzeichnet, dass** der Druck 30 bis 250 bar und/oder die Temperatur 70°C bis 140°C beträgt.

6. Verfahren gemäß einem der Ansprüche 1 bis 5, **dadurch gekennzeichnet, dass** im Aminonitrilgemisch 10 bis 70 Gew.-% DETMN enthalten ist.

7. Verfahren gemäß einem der Ansprüche 1 bis 6, **dadurch gekennzeichnet, dass** nach der Hydrierung TEPA, Triethylentetraamin (TETA) und gegebenenfalls weitere Ethylenamine, die als Nebenprodukte im jeweils erhaltenen Reaktionsprodukt enthalten sind, isoliert werden.

8. Verfahren gemäß einem der Ansprüche 1 bis 7, **dadurch gekennzeichnet, dass** DETDN und DETMN durch Umsetzung von Diethylentriamin (DETA) und Formaldehydcyanhydrin (FACH) hergestellt werden.

9. Verfahren gemäß Anspruch 8, **dadurch gekennzeichnet, dass** die Umsetzung von DETA und FACH in Gegenwart von Wasser erfolgt.

10. Verfahren gemäß Anspruch 8 oder 9, **dadurch gekennzeichnet, dass** direkt im Anschluss an die Herstellung von DETDN aus DETA und FACH die Hydrierung von DETDN erfolgt, wobei gegebenenfalls vor der Hydrierung eine Abreicherung von Wasser und/oder eine Leichtsiederabtrennung durchgeführt wird.

11. Verfahren gemäß Anspruch 10, **dadurch gekennzeichnet, dass** DETDN in einem Aminonitrilgemisch gemäß Anspruch 2 vorliegt.

12. Verfahren gemäß einem der Ansprüche 1 bis 11, **dadurch gekennzeichnet, dass** die Hydrierung in Gegenwart eines Additivs, insbesondere in Gegenwart von Ethylendiamin (EDA) oder Ammoniak, durchgeführt wird.

13. Aminonitril ausgewählt aus Diethylentriamindiacetonitril (DETDN) oder Diethylentriaminmonoacetonitril (DETMN).

14. Verfahren zur Herstellung eines Aminonitrils gemäß Anspruch 13, oder eines Gemisches davon, **dadurch gekennzeichnet, dass** Diethylentriamin (DETA) mit Formaldehyd und Blausäure (HCN) umgesetzt wird.

15. Verfahren gemäß Anspruch 14, **dadurch gekennzeichnet, dass** Formaldehyd und HCN zunächst zu Formaldehydcyanhydrin (FACH) und anschließend DETA mit FACH umgesetzt wird.

## Claims

1. A process for preparing tetraethylenepentamine (TEPA), which comprises hydrogenating diethylenetriaminediacetonitrile (DETDN) in the presence of a catalyst and a solvent.

2. The process according to claim 1, wherein DETDN is present in an amino nitrile mixture comprising i) at least 30% by weight of DETDN and ii) at least 5% by weight of diethylenetriaminemonoacetonitrile (DETMN).

3. The process according to claim 1 or 2, wherein a Raney catalyst, preferably a Raney nickel or Raney cobalt catalyst, in particular a skeletal Raney cobalt catalyst which has been obtained from a Co/Al alloy by leaching with aqueous alkali metal hydroxide solution and comprises at least one of the elements Fe, Ni or Cr as promoter, is used.

4. The process according to any of claims 1 to 3, wherein the solvent is water and/or an organic solvent, in particular tetrahydrofuran or methanol.

5. The process according to any of claims 1 to 4, wherein the pressure is from 30 to 250 bar and/or the temperature is from 70°C to 140°C.

6. The process according to any of claims 1 to 5, wherein the amino nitrile mixture comprises from 10 to 70% by weight of DETMN.

7. The process according to any of claims 1 to 6, wherein TEPA, triethylenetetramine (TETA) and optionally further ethylene amines which are comprised as by-products in the reaction product obtained in each case are isolated after the hydrogenation.

8. The process according to any of claims 1 to 7, wherein DETDN and DETMN are prepared by reaction of diethylenetriamine (DETA) and formaldehyde cyanohydrin (FACH).

9. The process according to claim 8, wherein the reaction of DETA and FACH is carried out in the presence of water.

10. The process according to claim 8 or 9, wherein the hydrogenation of DETDN is carried out directly after the preparation of DETDN from DETA and FACH, with removal of water and/or removal of low boilers optionally being carried out before the hydrogenation.

11. The process according to claim 10, wherein DETDN is present in an amino nitrile mixture according to claim 2.

12. The process according to any of claims 1 to 11, wherein the hydrogenation is carried out in the presence of an additive, in particular in the presence of ethylenediamine (EDA) or ammonia.

13. An amino nitrile selected from among diethylenetriaminediacetonitrile (DETDN) and diethylenetriaminemonoacetonitrile (DETMN).

14. The process for preparing an amino nitrile according to claim 13 or a mixture thereof, which comprises reacting diethylenetriamine (DETA) with formaldehyde and hydrocyanic acid (HCN).

15. The process according to claim 14, wherein formaldehyde and HCN are firstly reacted to form formaldehyde cyanohydrin (FACH) and DETA subsequently reacted with FACH.

## Revendications

1. Procédé pour la préparation de tétraéthylènepentamine (TEPA), dans lequel on soumet du diéthylènetriamine-diacétonitrile (DETDN) à une hydrogénation en présence d'un catalyseur et d'un solvant.

2. Procédé selon la revendication 1, dans lequel le DETDN est présent dans un mélange d'aminonitriles contenant i) au moins 30 % en poids de DETDN et ii) au moins 5 % en poids de diéthylènetriamine-monoacétonitrile (DETMN).

3. Procédé selon la revendication 1 ou 2, **caractérisé en ce qu'**on utilise un catalyseur de Raney, de préférence un catalyseur au nickel de Raney ou un catalyseur au cobalt de Raney, en particulier un catalyseur à squelette de cobalt de Raney, qui est obtenu à partir d'un alliage Co/Al par lixiviation avec une solution aqueuse d'hydroxyde de métal alcalin et qui contient en tant que promoteur au moins l'un des éléments Fe, Ni ou Cr.

4. Procédé selon l'une quelconque des revendications 1 à 3, **caractérisé en ce que** le solvant est l'eau et/ou un solvant organique, en particulier le tétrahydrofurane ou le méthanol.

5. Procédé selon l'une quelconque des revendications 1 à 4, **caractérisé en ce que** la pression est de 30 à 250 bars et/ou la température est de 70 °C à 140 °C.

6. Procédé selon l'une quelconque des revendications 1 à 5, **caractérisé en ce que** le mélange d'aminonitriles contient de 10 à 70 % en poids de DETMN.

7. Procédé selon l'une quelconque des revendications 1 à 6, **caractérisé en ce qu'**après l'hydrogénation on isole la TEPA, la triéthylène-tétramine (TETA) et éventuellement d'autres éthylèneamines qui sont contenues en tant que sous-produits dans le produit de réaction obtenu dans chaque cas.

8. Procédé selon l'une quelconque des revendications 1 à 7, **caractérisé en ce que** le DETDN et le DETMN sont préparés par mise en réaction de diéthylènetriamine (DETA) et formaldéhyde-cyanhydrine (FACH).

9. Procédé selon la revendication 8, **caractérisé en ce que** la réaction de DETA et FACH s'effectue en présence d'eau.

10. Procédé selon la revendication 8 ou 9, **caractérisé en ce que** l'hydrogénation du DETDN s'effectue directement à la suite de la préparation du DETDN à partir de DETA et FACH, une réduction de la teneur en eau et/ou une séparation de composés à bas point d'ébullition étant éventuellement effectuée(s) avant l'hydrogénation.

11. Procédé selon la revendication 10, **caractérisé en ce que** le DETDN est présent dans un mélange d'aminonitriles selon la revendication 2.

12. Procédé selon l'une quelconque des revendications 1 à 11, **caractérisé en ce qu'**on effectue l'hydrogénation en présence d'un additif, en particulier en présence d'éthylènediamine (EDA) ou d'ammoniac.

13. Aminonitrile choisi parmi le diéthylènetriamine-diacétonitrile (DETDN) ou le diéthylènetriamine-monoacétonitrile (DETMN).

14. Procédé pour la préparation d'un aminonitrile selon la revendication 13, ou d'un mélange de tels aminonitriles, **caractérisé en ce qu'**on fait réagir de la diéthylènetriamine (DETA) avec du formaldéhyde et de l'acide cyanhydrique (HCN).

15. Procédé selon la revendication 14, **caractérisé en ce qu'**on fait réagir d'abord du formaldéhyde et HCN pour obtenir de la formaldéhyde-cyanhydrine (FACH) et ensuite DETA avec FACH.
